# EUROPÄISCHE PATENTSCHRIFT

(11) **EP 2 068 801 B1**
(45) Veröffentlichungstag und Bekanntmachung des Hinweises auf die Patenterteilung: **16.03.2011**
(21) Anmeldenummer: 07801727.4
(22) Anmeldetag: 17.08.2007
(51) Int. Cl.: A61F 13/08

(54) **KOMPRESSIONSSTRUMPF, SET UND VERWENDUNG**
COMPRESSION STOCKING, SET, AND USE
BAS DE CONTENTION, ENSEMBLE ET UTILISATION

(30) Priorität: 02.10.2006 DE 102006048313
(43) Veröffentlichungstag der Anmeldung: 17.06.2009
(73) Patentinhaber: Paul Hartmann AG, 89522 Heidenheim (DE)
(72) Erfinder: VIRKUS, Antje, F-67000 Strasbourg (FR); BERNDT, Erik, 89542 Herbrechtingen (DE)
(74) Vertreter: Langöhrig, Angelika Beate
(86) Internationale Anmeldenummer: PCT/EP2007/007280
(87) Internationale Veröffentlichungsnummer: WO 2008/040414

(56) Entgegenhaltungen:
- DE-C- 968 307
- US-A- 2 694 395
- US-A- 6 142 961
- US-A1- 2004 193 084

## Beschreibung

Die Erfindung betrifft einen Kompressionsstrumpf aus einem elastischen Material mit einem an dem Kompressionsstrumpf befestigbaren Druckkörper. Kompressionsstrümpfe werden in der medizinischen Kompressionstherapie verwendet, um einen Ruhesowie einen Arbeitsdruck auf ein menschliches Bein aufzubringen. Unter dem Ruhedruck wird dabei der Druck verstanden, den der Strumpf als solcher auf das ruhende Bein aufbringt, wobei der Arbeitsdruck derjenige Druck ist, der beim Bewegen des Beins durch die Kontraktion der Muskeln und den Strumpf auf das Bein ausgeübt wird.
Dabei werden Kompressionsstrümpfe vielfach zur Therapie bei Venenerkrankungen auch im Rahmen von Veneneingriffen eingesetzt. Als Veneneingriffe kommen verschiedene Behandlungen in Frage, wobei Stripping eine sehr häufig durchgeführte Methode ist. Hierbei handelt es sich um die Entfernung einer geschädigten Vene, wobei zusätzlich Schaumsklerosierungen oder auch Laserinterventionen in Frage kommen. Je nach angewendeter Methode können unterschiedliche Nebenwirkungen und Komplikationen nach einem Veneneingriff auftreten. So wird vielfach seitens der Patienten über Schmerzen entlang der behandelten Vene bei einer Lasertherapie geklagt. Auch Blutergüsse kommen bei diesem Verfahren wie auch bei anderen Venenoperationsverfahren häufig vor. Bei der Sklerotherapie besteht die Gefahr, dass Farbeinlagerungen im Verlauf der Vene auftreten. Typische Folgen verschiedener Eingriffe sind Schmerzen, Ödembildung, Nachblutung, Hämatome, Indurationen und Pigmentierungen. Sämtliche dieser Nebenwirkungen können durch ausreichende Kompression zumindest stark verringert werden. Darüber hinaus ist eine Kompressionstherapie auch als notwendig angesehen worden, um den Erfolg der durchgeführten Maßnahmen langfristig zu sichern.

Im Stand der Technik ist dabei eine Vielzahl von Kompressionsstrümpfen bekannt, die zum Teil als Kniestrümpfe und zum Teil als Schenkelstrümpfe, die bis zum Schritt reichen, ausgelegt sein können. Darüber hinaus werden als Direktversorgung in den ersten Tage nach einer Operation vielfach Kompressionsbinden eingesetzt, die jedoch verhältnismäßig zeitaufwendig und kompliziert anzulegen sind, und die Gefahr von Fehlanwendungen bergen. Für die Nachversorgung derartiger Eingriffe oder Operationen wird in Deutschland ein Schenkelstrumpf der Kompressionsklasse 2 gemäß RAL-GZ 387 vom September 2000 eingesetzt.

Nachteilig bei den bekannten Lösungen ist, dass verschiedene Systeme für die Direktversorgung nach der OP sowie für die weitere Nachbehandlung eingesetzt werden müssen.

Des Weiteren ist es aus dem Stand der Technik bekannt, eine Kompressionstherapie durch den Einsatz von Druckkörpern zu ergänzen, die einen zusätzlichen Druck auf eine gewünschte Stelle oder auch Bereiche von zu behandelnden Gliedmaßen ausüben.

So offenbart beispielsweise die DE 19 55 539 A1 einen Kniestrumpf für den Unterschenkel mit einer aufblasbaren mehrteiligen Luftkammer, die zur Behandlung hypostatischer Erkrankungen eingesetzt werden kann. Es soll hier der Rückfluss von Blut aus den Beinen erleichtert werden. Hierzu ist eine Manschette vorgesehen, welche vom Fuß bis unterhalb der Kniekehle reicht und eine aufblasbare mehrteilige Luftkammer aus Gummi enthält, deren unterer Teil unter dem Fußsohlenbogen liegt und deren oberer Teil den zu behandelnden Partien des Unterschenkels zugeordnet ist. Hierdurch wird der Druck auf den gesamten Unterschenkel erhöht und so erreicht, dass das Blut leichter Richtung Herz abfließen kann.

Des Weiteren ist aus der DE 23 42 337 ein Massagestrumpf bekannt, bei dem Luft, die eine Massagefunktion ausüben soll, sich in einer oder mehreren Kammern befindet, wobei der Strumpf bis zum Knie hinauf reicht.

Aus der DE 34 47 916 A1 ist eine Vorrichtung zum Behandeln hypostatischer Beinleiden bekannt, wobei ein elastischer fest anliegender luftundurchlässiger Strumpf aus Gummi oder Kunststoff vorgesehen ist, in den durch ein in der Fußwölbung angebrachtes Gummiventil bei jedem Schritt durch die Belastung des Fußes eine kleine Luftmenge hineingepumpt wird. Diese Luft umgibt dann das Bein in dünner Schicht und steigt vom Fuß aus kontinuierlich nach oben und übt so eine Kompression auf das Bein aus.

DE 358 246 A1 offenbart einen Krampfadernverband mit elastischen Einlagen, wobei die Form und Größe der Einlagen so ist, dass sie die in jeder horizontalen Querschnittsfläche zwischen dem Umfang des kleinsten geometrischen Berührkreises des Beins und dem Bein selbst freibleibenden hohlen Stelle ausfüllen, damit in allen Punkten des umspannten Querschnitts ein möglichst gleichmäßiger Flächendruck hervorgerufen wird.

Des Weiteren ist aus der EP 1 391 190 B1 ein Kompressionsstrumpf mit einem Kompressionskörper bekannt, der im Bereich des Kniegelenks angeordnet ist, um die dortigen Gelenkweichteile mit Druck zu beaufschlagen.

US 20040193084 offenbart auch ein Kompressionprodukt.

Ausgehend von diesem Stand der Technik ist es nun Aufgabe der vorliegenden Erfindung, einen Kompressionsstrump des Anspruchs 1 sowie ein Set des Anspruchs 12 bestehend aus einen Kompressionsstrumpf und einem Druckkörper bereitzustellen, der einen Einsatz sowohl als Direktversorgung nach Venenoperationen als auch für die weitere Nachbehandlung ermöglicht.

Die Erfindung löst diese Aufgabe durch einen Kompressionsstrumpf des Anspruchs 1 aus einem elastischen Material mit einem an dem Kompressionsstrumpf befestigbaren Druckkörper, wobei der Kompressionsstrumpf ein Schenkelstrumpf ist und an seinem an einer Oberschenkelinnenseite eines Trägers zu liegen kommenden Bereich eine Tasche aufweist, in die der Druckkörper entnehmbar einbringbar ist.

Unter einem Schenkelstrumpf ist dabei hinsichtlich der Länge des Strumpfes am Bein eines Trägers ein solcher Strumpf zu verstehen, wie er gemäß der Richtlinie RAL-GZ 387, September 2000, definiert ist und sich aus dieser entnehmen lässt.

Dabei soll vorgesehen sein, dass an der Oberschenkelinnenseite des Kompressionsstrumpfes eine Tasche zur Aufnahme eines Druckkörpers vorgesehen ist, wobei der Druckkörper einen lediglich lokalen Druck auf die Oberschenkelinnenseite aufbringt.

In diesem Bereich ist die große Saphenavene (Vena saphena magna) angeordnet, die in aller Regel durch Venenbehandlungen betroffen ist. Durch die Kompressionsaufbringung auf diesen Bereich kann so der Therapieerfolg in den ersten zwei bis fünf Tagen nach der Operation gesichert werden. Im weiteren Verlauf kann dann der Druckkörper aus der Tasche entnommen werden und der Kompressionsstrumpf kann als normaler Kompressionsstrumpf der entsprechenden Kompressionsklasse für die nächsten Wochen weitergetragen werden. Es ist somit nicht länger erforderlich, verschiedene Behandlungssysteme, wie Kompressionsbinden, kombiniert mit einem späteren Tragen eines Kompressionsstrumpfes zu verwenden. Die finanziellen Mittel zur Nachversorgung nach einer Venen-OP können so erheblich vermindert werden.

Es kann dabei vorgesehen sein, dass die Tasche hinsichtlich ihrer Größe an den Druckkörper angepasst ist. D. h., die Tasche ist um so viel größer als der Druckkörper, dass dieser problemlos in die Tasche eingeschoben und aus dieser entnommen werden kann, ohne jedoch ein Verrutschen des Druckkörpers aufgrund eines zu großen Spiels desselben in der Tasche zu ermöglichen. Insbesondere ist dabei vorgesehen, dass die Tasche auf der einen Seite aus dem Strumpf selbst und auf der anderen Seite aus einem zweiten Materialabschnitt besteht, der an dem Strumpf auf der im angelegten Zustand innen liegenden Seite des Strumpfes angeordnet insbesondere angenäht ist. Insbesondere besteht dieser zweite Materialabschnitt aus einem Gestrick, Gewirk oder Gewebe, das elastischer ist als der Strumpf selbst. Somit bildet die Tasche ein Haltesystem ohne die Kompressionseigenschaften des Strumpfes zu verändern oder zumindest nicht wesentlich zu verändern.

Nach einer besonders bevorzugten Ausführungsform kann vorgesehen sein, dass der Druckkörper befüllbar und entleerbar ist, insbesondere mit einem Gas oder einer Flüssigkeit, vorzugsweise Luft oder Wasser. Insbesondere ist vorgesehen, dass der Druckkörper stufenlos befüllbar und entleerbar ist. Auf diese Weise wird erreicht, dass durch den Patienten oder den Arzt der Druck im Druckkörper entsprechend der Situation des jeweiligen Patienten optimal eingestellt werden kann. D. h., der Druck kann durch die Menge des in den Druckkörper eingefüllten Mediums entsprechend so eingestellt werden, dass auf der einen Seite eine ausreichende Kompression auf der zu behandelnden Region am Oberschenkel erreicht wird und auf der anderen Seite die Kompression bzw. der Druck auf die entsprechende Stelle dem Patienten beim Tragen keine Schmerzen bereitet. Es kann hierzu vorgesehen sein, dass der Druckkörper ein Ventil besitzt, wobei er beispielsweise mit Luft über einen entsprechenden Blasebalg, der mit dem Druckkörper gekoppelt werden kann, befüllbar ist. Das Ventil kann hierbei ein übliches Ventil zur Befüllung von Hohlkörpern mit Luft sein. Ein solches System bietet gegenüber der Beliebigkeit der Verwendung z. B. von gerollten Kompressen als Druckkörper, die unter eine Binde gelegt werden, Vorteile hinsichtlich der Druckdefinierung und durch die Einbringung in die Tasche kann die Position des Druckkörpers leichter fixiert werden.

Insbesondere hat sich gezeigt, dass ein Druckkörper mit einer Auflagefläche von mindestens 50 cm², insbesondere von mindestens 80 cm² und ganz besonders von mindestens 120 cm² einen Druck auf den zu behandelnden Oberschenkelbereich aufbringt, der die oben geschilderten Folgen nach einem Eingriff nachhaltig entgegenwirkt.

Bei der Ausgestaltung des Druckkörpers ist eine Reihe von verschiedenen Konstruktionen möglich. So kann es sich bei dem Druckkörper vorteilhafter Weise um ein Einkammersystem handeln, dass über ein Ventil befüllbar ist. Es ist jedoch auch möglich ein Druckkörper zu verwenden, der ein Zwei- oder Mehrkammersystem umfasst.

Neben einem entleer- oder befüllbaren Druckkörper sind jedoch auch Druckkörper denkbar, die ein festes Volumen besitzen, wobei diese insbesondere in gewissem Maße elastisch verformbar sein können. Diese Druckkörper können dann auch eine Hülle aufweisen, die mit einem Feststoff oder beispielsweise einem Schaum oder einem Elastomermaterial befüllt ist.

So kann hierbei insbesondere vorgesehen sein, dass der Druckkörper eine längliche Form besitzt, wobei dessen Längsachse am Oberschenkel eines Trägers von proximal nach distal verläuft. Dabei kann der Druckkörper insbesondere auf dem Bereich der Vena saphena magna aufliegen, da hier eine Kompression nach beispielsweise einem Venenstripping oder einer Laserintervention besonders erwünscht ist. Der Druckkörper kann eine solche Länge haben, dass er sich im Wesentlichen von der Oberkante des Strumpfes bzw. unterhalb eines entsprechenden Bündchens des Strumpfes bis in den Oberschenkelbereich bis zum Knie hinabzieht, so dass im Wesentlichen der gesamte Oberschenkel an seiner Innenseite durch den Druckkörper mit Druck beaufschlagt wird.

Auf diese Weise kann der Druck besonders gleichmäßig über den gesamten notwendigen Kompressionsbereich nach Venenoperationen aufgebracht werden, was zum einen eine gute Heilung ermöglicht und zum anderen den entsprechenden Druckkörper in seiner Tragbarkeit für den Patienten verbessert.

Dabei führt der Einsatz eines Druckkörpers zunächst auch dazu, dass der Druck auf die Oberschenkelinnenseite durch einen Kompressionsstrumpf besser aufgebracht werden kann, da durch die Abflachung der Oberschenkelinnenseite im Vergleich zum übrigen Beinabschnitt hier die Druckaufbringung nicht im gleichen Maße wie in den übrigen Bereichen erfolgen kann. So ist eine Aufbringung eines Drucks auf einen Körper in gleichmäßiger Form nur dann möglich, wenn dieser das Idealprofil einer Kreisform besitzt. Besitzt ein Körper eine Abflachung an einer Stelle, so wird auf diesen Bereich nur ein geringerer Druck im Vergleich zu dem übrigen stärker gewölbten Bereich aufgebracht. Durch die Verwendung eines Druckkörpers auf den abgeflachten Bereich einer Oberschenkelinnenseite kann dieser Druck nun erhöht werden in einem Maß, wie er zur Behandlung von nachoperativen Problemen bei Venenoperationen notwendig ist.

Es kann dabei vorgesehen sein, dass das elastische Material ein Gewebe, Gewirke oder Gestricke, vorzugsweise ein Rundgestricke, ist. Dabei kann es vorgesehen sein, dass es sich bei den verwendeten Materialien um solche handelt, die aus Fäden bestehen, bei denen ein elastischer Kernfaden mit einem Baumwollfasern enthaltenden Garn, insbesondere einem Stapelfasergarn, als Umwindefaden umwickelt ist. Dabei kann besonders vorteilhaft vorgesehen sein, dass als Kernfaden ein Elastankern, beispielsweise ein Kern aus Lycra ®, verwendet wird, wobei dieser Kern einfach oder doppelt oder mehrfach umwunden sein kann und wobei das zum Umwinden verwendete Garnmaterial zum einen ein Multifilamentgarn aus Polyamid sein kann und zum anderen zum Umwinden sowohl ein Multifilamentgarn aus Polyamid als auch ein Stapelfasergarn verwendet werden kann.

Dabei ist es vorteilhaft, wenn das Stapelfasergarn 70 bis 90 % Baumwolle und 10 bis 30 % eines Algen enthaltenden Zellulosematerials beinhaltet. Als Algenmaterial enthaltende Zellulosefasern können insbesondere Seacell-Fasern ® (SeaCell GmbH, Rudolstadt, Deutschland) eingesetzt werden. Bei diesen Fasern ist in die Zellulosefaser Algenmaterial inkorporiert. Die Algen liegen dabei in Partikelform gleichmäßig innerhalb der Zellulosefaser vor.

Dabei können folgende Materialien bevorzugt verwendet werden:
Strickfaden Schaft und Fuß:
   78 dtex EL Lycra ® (Kern) - doppelt umwunden mit 26f28/1 dtex PA 6.6 text. (Mikrofasergarn)
Einlegefaden Schaft und Fuß:
   570 dtex EL Lycra ® (Kern) - doppelt umwunden mit
      (i) 44f13/1 dtex PA 6.6 text. und
      (ii) Stapelfasergarn Nm 170/1 (Seacell ®)
Pendelferse:
   44 dtex Lycra ® (Kern) - doppelt umwunden mit 42f46/1
   PA 6.6 text. Tactel ® (Mikrofaser) mit Aufplattierung mit
   13f14/2 dtex PA 6.6 text. (Mikrofaser)
wobei die Fußspitze offen mit abgesäumtem Bund ausgestattet ist.

Nach einer alternativen Ausgestaltung kann als Strickfaden für die Länge, den Fuß, die Ferse und die Spitze ein 44 dtex Lycra ®-Kern doppelt umwunden mit 42f46/1 dtex Tactel ® Mikrofaser verwendet werden. In dieser Ausgestaltung wird ein Einlegefaden für die Länge und den Fuß aus 395 dtex Lycra ®-Kern - doppelt umwunden mit 26f28/1 dtex Tactel ® (Mikrofaser) verwendet werden. Die Ferse und die geschlossene Spitze weisen keinen Einlegefaden auf.

In beiden Fällen wird ein Schussfaden 1:1 alternierend in jeder Reihe eingesetzt.

Für die Tasche kann als Nahtmaterial und als Nahttyp eine 2 Nadel Flatseamer Naht; Nähfaden PA 2x (gezwirnt) verwendet werden, wobei als Strickfäden für die Tasche folgendes eingesetzt werden kann:
1. Reihe: 78 dtex Lycra ® Kern einfach umwunden mit 67f20/1 PA 6.6 text.
2. Reihe: 33f10/2 PA 6.6 text.

Die Spitze eines Fußbereichs des Kompressionsstrumpfes kann dann glatt und geschlossen oder offen aufgebaut sein.

Bei einem erfindungsgemäßen Kompressionsstrumpf handelt es sich um einen Kompressionsstrumpf, der ohne Druckkörper einen Druckverlauf nach RAL-GZ 387 oder Referentiel techniques des ortheses elastiques de contention des membres (ASQUAL) Réviosn Nr. 5, Seite 15/23 aufweist.

Aus den Prüfbestimmungen der RAL-Norm kann entnommen werden, wie der Druck eines Kompressionsstrumpfes auf ein Bein zu bestimmen ist. Dabei liegen vier Kompressionsklassen vor, wobei die Restdruckverhältnisse der Kompressionsklasse II an der Messstelle B-100 %, an der Messstelle B1 70 bis 100 %, an der Messstelle C 50 bis 80 % und an den Messstellen F oder G 20 bis 50 % betragen. Aus der französischen Referentiel techniques des ortheses elastiques de contention des membres sind ebenfalls vier Kompressionsklassen sowie eine Sonderklasse mit einem niedrigen Kompressionsdruck bekannt. Die Kompression wird hier an der Fessel, was etwa dem Punkt B entspricht, gemessen oder definiert nach der Methode MR 028 (Annex 8).

Des Weiteren kann besonders bevorzugt vorgesehen sein, dass der Druckkörper einen solchen Druck auf das Bein unterhalb des Druckkörpers ausübt, dass hier ein Druck von 15 bis 60 mm Hg und insbesondere 20 bis 45 mm Hg über dem Druck des Kompressionsstrumpfes an dieser Stelle ohne Druckkörper ausgeübt wird. Der Druck, der durch den Druckkörper ausgeübt wird, addiert sich dann zusammen mit dem Druck, der durch den Kompressionsstrumpf bereits auf das Bein resultiert. Es kann dabei besonders bevorzugt vorgesehen sein, dass der Gesamtdruck im Bereich des Druckkörpers wenigstens 25 mm Hg, aufgebracht durch den Kompressionsstrumpf und den Druckkörper, beträgt. Bevorzugt liegt jedoch ein höherer Druck an dieser Stelle vor. Der Druck des Druckkörpers wird dabei durch den Füllungsgrad des Druckkörpers bestimmt und sollte nicht mehr als 70 mm Hg betragen.

Besonders bevorzugt ist, dass der Druck des Kompressionsstrumpfes im Bereich außerhalb des Druckkörpers im Wesentlichen unabhängig von der Einbringung oder Befüllung des Druckkörpers ist. Hierbei soll unter unabhängig verstanden werden, dass der Druck außerhalb des Druckkörpers über den übrigen Umfang des Oberschenkels eines Trägers um höchstens 3 mm Hg zunimmt. Vorzugsweise nimmt der Druck über den übrigen Umfang um höchstens 2 mm Hg und besonders bevorzugt um höchstens 1 mm Hg zu. Auf diese Weise kann erreicht werden, dass der Druck lokal unter dem Druckkörper auf die zu behandelnde und zu komprimierende Stelle aufgebracht werden kann. Eine Erhöhung der Kompression in den übrigen Bereichen tritt annähernd nicht auf, so dass der erwünschte Druckverlauf gemäß den zuvor zitierten Normein eingehalten werden kann und so die Funktion des Kompressionsstrumpfes, nämlich eine erhöhte Kompression von der Fessel her abnehmend zum Oberschenkel, weiter aufrechterhalten bleibt. Auf diese Weise kann vermieden werden, dass eine Stauung des rückfließenden Blutes in der Extremität durch die Druckerhöhung im Bereich des Oberschenkels erfolgt.

Es kann des Weiteren besonders vorteilhaft vorgesehen sein, dass der Kompressionsstrumpf am Oberschenkel durch ein an der Innenseite eines Bündchens angeordnetes Haftband gegen Rutschen nach distal gesichert ist, wobei das Haftband eine die Haftung zwischen dem Bein des Trägers und dem Kompressionsstrumpf erhöhende Beschichtung aufweist. Es kann hierbei insbesondere eine Silikonbeschichtung oder auch eine andere Beschichtung vorgesehen sein, wie sie üblicherweise bei Kompressionsstrümpfen oder auch anderen Strümpfen, die halterlos getragen werden, vorgesehen ist, und eine Haftung vorzugsweise dergestalt, dass das Haftband auf der Haut des Trägers haftet, jedoch ein Verkleben mit übrigen Teilen vermieden wird, vorgesehen ist. Die Beschichtung kann dabei vorzugsweise in Gittern oder in Umfangsrichtung verlaufenden Streifen oder kalottenförmig aufgebracht sein. Auf diese Weise wird erreicht, dass die elastischen Eigenschaften des Strumpfes hinreichend erhalten bleiben und darüber hinaus eine Luft- und Wasserdampfdurchlässigkeit in diesem Bereich sichergestellt werden kann.

Nach einer besonders bevorzugten Ausführungsform kann vorgesehen sein, dass ein von distal nach proximal schräger Verlauf an der Oberschenkelinnen- und/oder -außenseite von posterior nach anterior des Bündchens vorgesehen ist, wobei dieser Verlauf gekrümmt oder ungekrümmt sein kann. Dies bedeutet, dass bei der Betrachtung eines Trägers eines entsprechenden Strumpfes von der Seite das Bündchen von dessen Rücken zu dessen Bauchseite schräg nach oben verläuft. Der Verlauf ist dabei von unterhalb des Überganges von Oberschenkel zur Pobacke, der generell niedriger angeordnet ist in Bezug auf einen am Boden stehenden Träger eines entsprechenden Strumpfes als die Gehfalte zwischen Oberschenkel und Rumpf. Dieser Verlauf kann dabei geschwungen oder ungeschwungen sein, wobei insbesondere der geschwungene Verlauf mit einem Bogen im Bereich der halben Schenkelbreite eine besonders anatomische Gestaltung besitzt. Das Bündchen besitzt dann in einer Seitenansicht eines Trägers z.B. eine S-Form. Dabei können die Bündchenverläufe an der Schenkelinnen- und der Schenkelaußenseite des selben Strumpfes voneinander verschieden sein. Eine entsprechende Ausgestaltung der Bündchenform kann auch bei anderen Kompressionsstrümpfen unabhängig von den übrigen Merkmalen, wie der Einbringung der Tasche und des Druckkörpers, vorteilhaft vorgesehen sein.

Die Erfindung betrifft darüber hinaus ein Set bestehend aus einem Kompressionsstrumpf aus einem elastischen Material, wobei der Kompressionsstrumpf ein Schenkelstrumpf ist und an seinem an einer Oberschenkelinnenseite eines Trägers zu liegen kommenden Bereich eine Tasche aufweist sowie einem an die Tasche angepassten Druckkörper, der in die Tasche entnehmbar einbringbar ist. Das Set besitzt somit eine doppelte Anwendungsfunktion. So kann der Strumpf mit eingeschobenem Druckkörper, der stufenlos befüll- und entleerbar ist, zur direkten Nachbehandlung nach einer Venenoperation eingesetzt werden, wobei er hier einen zusätzlichen Kompressionsdruck im Bereich der Vena saphena magna aufzubringen vermag. Nach der ersten unmittelbaren Versorgung nach der Operation kann dann zur weiteren Nachbehandlung der Druckkörper aus der Tasche entnommen werden und der Strumpf kann als herkömmlicher Kompressionsstrumpf weiter getragen werden und so den Behandlungserfolg nachhaltig unterstützen.

Bei dem Kompressionsstrumpf kann es sich generell, sowohl bei dem vorstehend beschriebenen als auch bei dem Kompressionsstrumpf des Sets, um einen Kompressionsstrumpf der Kompressionsklasse II bzw. 2 handeln. Dies gilt sowohl für die Kompressionsklassen nach Norm RAL-GZ 387 als auch nach der Norm Referentiel techniques des ortheses elastiques de contention des membres, die zuvor angegeben worden ist. Schließlich betrifft die Erfindung eine Verwendung eines Kompressionsstrumpfes der vorstehend beschriebenen Art sowie eines Sets der vorstehend beschriebenen Art zur postoperativen Behandlung von Venenerkrankungen der Vena saphena magna, indem der Kompressionsstrumpf so getragen wird, dass der Druckkörper auf dem Bereich der Vena saphena magna an der Oberschenkeninnenseite zu liegen kommt. Dabei kann der Kompressionsstrumpf oder das Set vorzugsweise so verwendet werden, dass nach einer definierten Tragezeit mit Druckkörper der Druckkörper entfernt werden kann und der Kompressionsstrumpf ohne Druckkörper weiter getragen wird.

Im Folgenden soll anhand einer Zeichnung die Erfindung näher erläutert werden. Dabei zeigen:
- Figur 1: einen erfindungsgemäßen Kompressionsstrumpf;
- Figur 2: einen Schnitt entlang der Linie A-A ohne Druckkörper;
- Figur 3a und 3b: einen Schnitt entlang der Linie A-A mit einem Druckkörper;
- Figuren 4 und 5: Gestaltungen des Bündchens eines erfindungsgemäßen Kompressionsstrumpfs;
- Figuren 6a - 6d: Ausgestaltung eines Druckkörpers in verschiedenen Ansichten
- Figur 7: ein Legebild eines Teils eines Schaftbereiches eines erfindungsgemäßen Kompressionsstrumpfs und
- Figur 8: ein menschliches Bein von der Schenkelinnenseite.

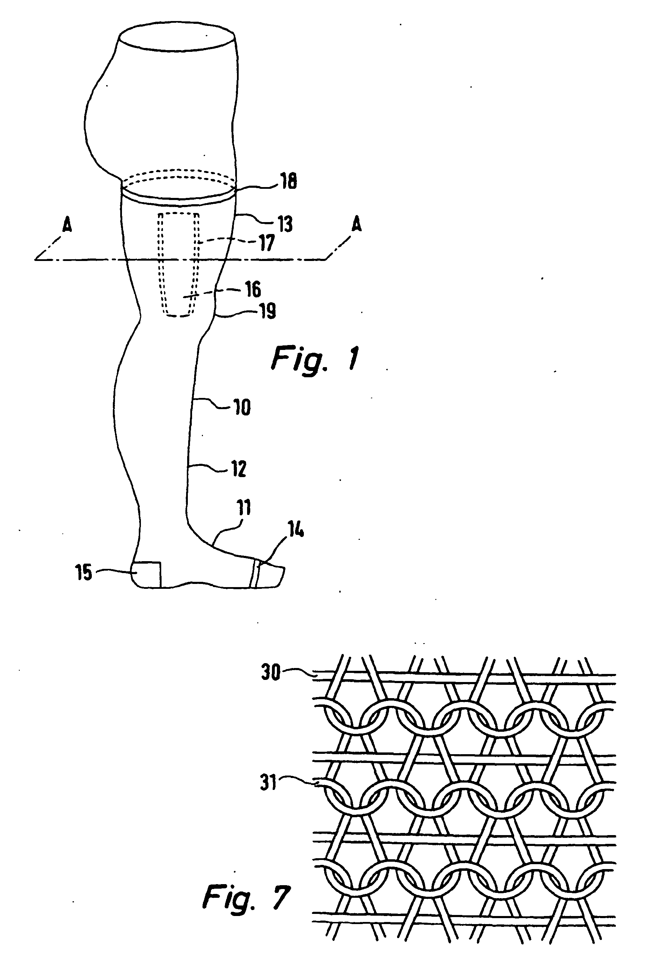

Figur 1 zeigt einen erfindungsgemäßen Kompressionsstrumpf, der als Schenkelstrumpf (ein sogenannter AG-Strumpf bezugnehmend auf die Messbezugspunkte gemäß der Norm RAL-GZ 387) gemäß der Norm RAL-GZ 387 ausgebildet ist. Dabei liegt der Punkt A im Bereich der Zehe und der Punkt G im Bereich des Übergangs des Oberschenkels in den Rumpf.

Der Kompressionsstrumpf ist hierbei mit dem Bezugszeichen 10 versehen. Er lässt sich in einen Fußbereich 11, einen Unterschenkelbereich 12 sowie einen Oberschenkelbereich 13 gliedern. Darüber hinaus ist ein Kniebereich vorgesehen, der mit 19 bezeichnet ist.

Ferner ist ein Fersenbereich 15 vorgesehen, der als Pendelferse gestaltet ist, die von einem Druckentlastungsbereich umschlossen sein kann (in Fig 1 nicht gezeigt). Die Pendelferse ist nahtlos an den übrigen Fußbereich 11 angeformt. Im Bereich der Zehe ist ein Bündchen 14 mit offener Spitze vorgesehen, wobei die Spitze grundsätzlich auch geschlossen ausgebildet sein kann. Bei dem Kompressionsstrumpf 10 handelt es sich um einen Strumpf aus einem elastischen Gestrick, das vorzugsweise nahtlos als Rundgestrick ausgeführt ist.

In Figur 1 ist ein Bein von der Innenseite her gezeigt. Im Bereich des Oberschenkels ist eine Tasche 16 zu erkennen, in die ein später zu beschreibender Druckkörper von proximal nach distal eingeschoben werden kann. Hierzu weist die Tasche 16 an ihrem proximalen Ende eine Öffnung auf und ist an ihrem distalen Ende geschlossen und zur leichteren Entnehmbarkeit eines Druckkörpers zum distalen Ende hin konisch zulaufend gestaltet. Die Tasche kann dabei alternativ auch unten offen gestaltet sein, insbesondere, sofern sie konisch zulaufend ist, da so ein Herausfallen des Druckkörpers nach distal verhindert werden kann.

Die Tasche erstreckt sich dabei über den gesamten Oberschenkelinnenbereich eines Trägers, wobei das obere Ende der Tasche 16 so viel von einem Bündchen 18 beabstandet ist, dass auch bei eingeschobenem Druckkörper das Bündchen sicher gegen die Beininnenseite eines Trägers anliegt. Nach unten erstreckt sich die Tasche 16 bis unmittelbar über oder sogar bis in den Kniebereich 19 hinein.

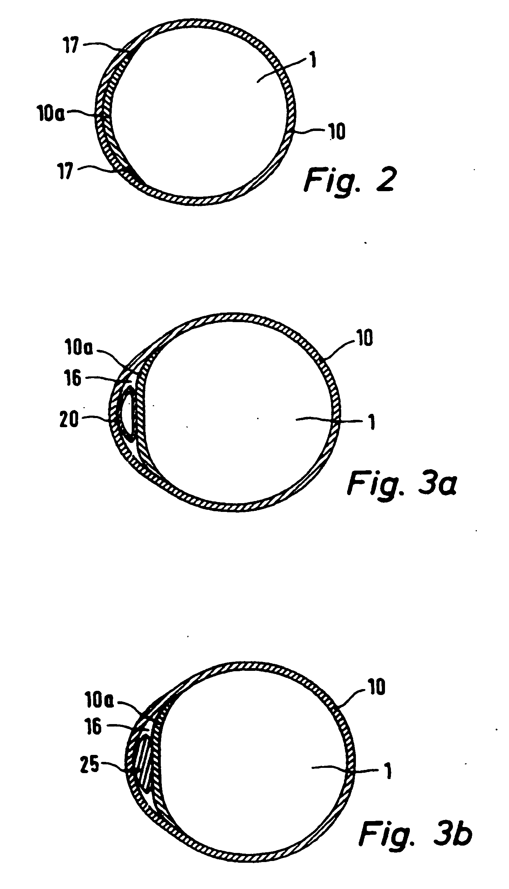

In Figuren 2, 3a und 3b ist nun ein Schnitt entlang der Linie A-A durch ein Bein gemäß Figur 1 gezeigt, wobei hier jeweils ein linkes Bein zu sehen ist. Die Blickrichtung ist in Figuren 2, 3a und 3b von proximal nach distal. Das Bein ist mit dem Bezugszeichen 1 gekennzeichnet, wobei die Tasche 16 durch den Strumpf 10 und einem zweiten Materialabschnitt 10a, der über zwei Nähte 17 mit dem Kompressionsstrumpf 10 verbunden ist, gebildet wird. Durch Einlegen eines Druckkörpers 20 oder 25, wie er in Figur 3a und 3b dargestellt, kann nun der radiale Druck unterhalb des Druckkörpers 20 oder 25 auf die Oberschenkelinnenseite eines Beins eines Trägers erhöht werden. Der Druckkörper 20 oder 25 kann wie in Figur 3b gezeigt ein Vollkörper-Silikonpelotte oder wie in Figur 3a gezeigt eine mit Luft befüllbarer Druckkörper sein. Dabei kommt der Druckkörper 20 oder 25, wie in Figur 1 ersehen werden kann, im Bereich der Vena saphena magna zu liegen und erhöht lokal in diesem Bereich den Druck, der vielfach im Rahmen von Venenoperationen betroffen ist.

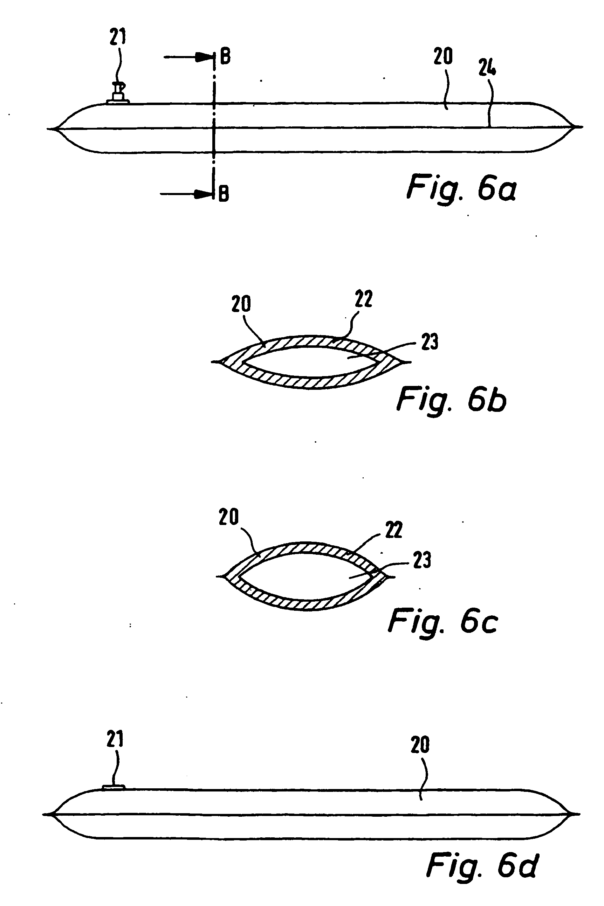

Mit Figuren Figur 6a - 6d ist ein befüllbarer und entleerbarer Druckkörper 20 dargestellt, wobei mit Figuren 6b und 6c ein Querschnitt des Druckkörpers 20 aus Figur 6a dargestellt ist. Dieser Druckkörper 20 ist dabei eine mit Luft befüllbare Einkammerpelotte, die ein Ein- und Auslassventil 21 aufweist, über das der Füllungsgrad geregelt werden kann; vgl. Fig. 6b geringerer Füllstand im Vergleich zu Figur 6c höherer Füllstand. Der Füllungsgrad wird dabei so eingestellt, dass ein ausreichender Druck auf die Oberschenkelinnenseite aufgebracht wird und gleichzeitig der Druck für den Patienten jedoch nicht schmerzhaft ist.

Als Druckkörper 20 wird ein länglicher Druckkörper eingesetzt, der im Wesentlichen der Form der Tasche 16 angepasst ist. Insbesondere handelt es sich hierbei um zwei ultraschallverschweißte PVC-Folien 22 mit eingeschweißtem Ventil 21 aus PVC mit Rückschlagventil und Verschlussklappe, wie es im Stand der Technik bekannt ist. Ein derartiger Druckkörper kann dann über einen Schlauch an einen Blasebalg angeschlossen werden, um den Druck im Druckkörper aufzubauen. Nach Entfernen des Schauchs kann die Verschlusskappe verschlossen werden. Der Druck im Druckkörper kann nach Entfernen der Verschlusskappe durch Drücken des Ventils gemindert bzw. ganz abgebaut werden. Das Ventil 21 kann durch Eindrücken in den Druckkörper 20 versenkt werden und stört dann beim Tragen des Strumpfes mit Druckkörper 20 nicht mehr (vgl. Fig. 6d). Bei unbefülltem Druckkörper kann dieser als Maße eine Länge von 25 bis 45, vorzugsweise 27 cm und eine Breite von 5 bis 9 cm, vorzugsweise 7 cm aufweisen.

Bei dem gezeigten Kompressionsstrumpf handelt es sich dabei um einen Kompressionsstrumpf der Klasse II nach RAL mit einem Druck von 23 bis 32 mg Hg an der Messstelle B, wobei die Restdruckverhältnisse für die Kompressionsklasse II für die Messstelle B1 70 bis 100 %, für die Messstelle C 70 bis 80 % und für die Messstellen F oder G 20 bis 50 % betragen. Die Messpunkte entsprechen dabei folgenden Stellen am menschlichen Bein: Messstelle B entspricht einem Punkt am menschlichen Unterschenkel, der unmittelbar über dem Knöchel an dem Punkt mit dem geringsten Umfang des Unterschenkels liegt. Die Messstelle B1 entspricht einem Punkt am menschlichen Unterschenkel, der im vorgegebenen Abstand darüber, also proximal, beim Übergang der Achillessehne zur Wade angeordnet ist. Weitere Punkte sind beispielsweise der Punkt C, nämlich der größte Umfang der Wade, sowie der Punkt D, der zwei Fingerbreit unterhalb der Kniekehle liegt. Dabei entspricht die Messstelle F einem Punkt am menschlichen Oberschenkel, der in der Mitte zwischen dem Poplitealpunkt des Knies und des halben Oberschenkels liegt. Alternativ kann es sich um einen Strumpf nach einer Kompressionsklasse 2 mit einem Druck von 20,1 bis 27 hPA (15 bis 20 mm Hg handeln, wobei dieser Druck den Druck etwa am Punkt B oder definiert nach der Methode MR 028 (Annex 8) darstellt.

Hieraus ergeben sich Druckwerte an Punkten F bis G von etwa 10-11 mm Hg gemäß der RAL-Norm RAL GZ 387. Bei einem Strumpf gemäß der französischen Kompressionsklasse 2 liegt im anderen Fall die Degressivität zwischen Fessel und Schenkel bei 70 %. D. h. ein Strumpf gemäß der französischen Norm würde an den Punkten F bis G einen Druck von ca. 7 mm Hg aufweisen, wobei der deutsche Strumpf einen Druck von ca. 10 bis 11 mm Hg aufweist. Misst man nun den durch einen Druckkörper zusätzlich aufgebrachten Druck, so entsteht hier je nach Füllungsgrad des Druckkörpers ein Druck von 25 bis 45 mm Hg, wobei ein minimaler Druck von 30 mm Hg als Gesamtdruck entstehend aus dem Druck des Strumpfes sowie des Druckkörpers erforderlich ist, um einen therapeutischen Effekt zu erzielen.

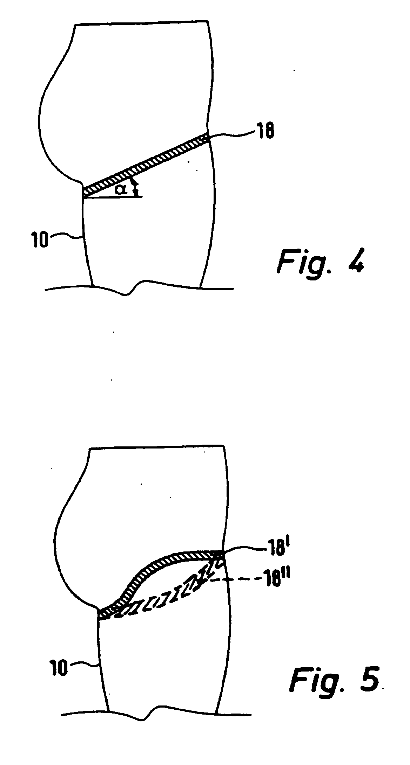

Die Figuren 4 und 5 zeigen nun verschiedene Bündchengestaltung, wobei das Bündchen 18 eines Strumpfes 10 in Figur 4 einen von posterior nach anterior von distal nach proximal schrägen Verlauf unter einem Winkel α zur Horizontalen aufweist. Mit Figur 5 ist ein Bündchen 18 dargestellt, das gegenüber Figur 4 prinzipiell denselben Verlauf aufweist, jedoch gekrümmt ausgeformt ist und als anatomisch angepassten Verlauf bezeichnet werden kann. Dabei ist mit 18' der Verlauf des Bündchens an der Schenkelaußenseite und mit 18" an der Schenkelinnenseite des Strumpfes 10 bezeichnet. Die Bündchenverläufe 18' und 18" an der Schenkelinnen- bzw. der Schenkelaußenseite des selben Strumpfes können voneinander verschieden sein. Durch diese Gestaltung wird erreicht, dass auch in der Bewegung das Bündchen eine sichere Haftung besitzt und insbesondere im Übergangsbereich zwischen Oberschenkel und Pobacke bzw. im vorderen Bereich im Übergang zwischen Oberschenkel und Rumpf so zu liegen kommt, dass es nicht zu einem Umschlagen des Bündchens bei einer Bewegung des Trägers kommt.

Figur 7 zeigt nun ein Legebild eines Strumpfes, wobei jeweils ein Schussfaden 1:1 alternierend in jeder Reihe angeordnet ist. Der Schussfaden ist hier mit dem Bezugszeichen 30 versehen. Zusätzlich wird ein Strickfaden 31 verwendet.

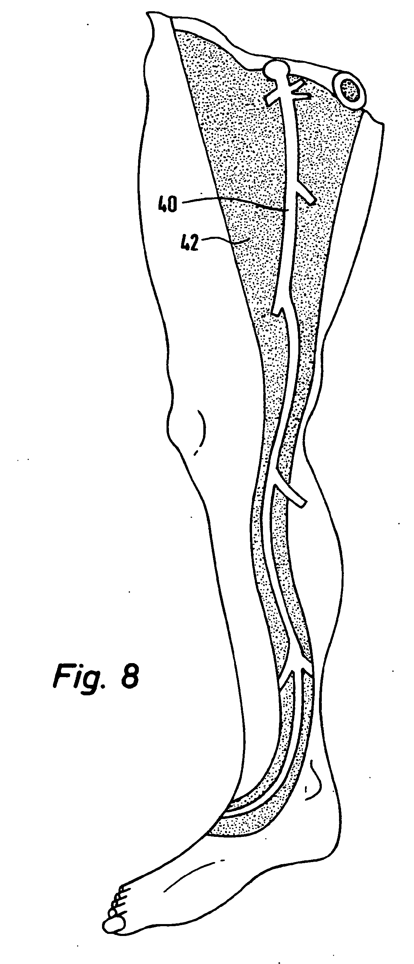

Figur 8 zeigt ein menschliches Bein von der Schenkelinnenseite, wobei hier die Vena saphena magna dargestellt ist, die bei Venenoperationen in aller Regel entweder durch Stripping entfernt oder z. B. durch Schaumsklerosierungen gefüllt wird. Die Vena saphena magna ist hierbei mit dem Bezugszeichen 40 versehen. Mit dem Bezugszeichen 42 ist dabei der Bereich gekennzeichnet, in dem die Vena saphena magna üblicherweise im Körper angeordnet ist. In diesem Bereich der Innenseite des Oberschenkels kann nun durch den zusätzlichen Druckkörper 20, der in die Tasche 16 eingeschoben werden kann, ein Druck unmittelbar nach einer Operation aufgebracht werden. Nach einigen, ca. drei bis fünf, Tagen Tragezeit mit Druckkörper 20 kann dieser dann entfernt werden und der Kompressionsstrumpf 10 kann für die übrige Behandlungszeit oder auch dauerhaft ohne den Druckkörper weiter getragen werden. Dabei wird der Druck unterhalb des Druckkörpers 20 lediglich lokal erhöht. Eine Erhöhung des Drucks im übrigen Umfangsbereich erfolgt hierbei um maximal 2 mm Hg, so dass der Kompressionsstrumpf 10 außerhalb des Bereichs, der unmittelbar unter dem Druckkörper 20 liegt, die Druckverhältnisse eines herkömmlichen RAL-Strumpfes bzw. eines Strumpfes gemäß der französischen Norm aufweist.

Auf diese Weise kann erzielt werden, dass zwei Behandlungsabschnitte, nämlich der unmittelbare Post-OP-Bereich und der Bereich der Nachbehandlung, durch einen Kompressionsstrumpf abgedeckt werden können, so dass eine ökonomische Variante zur Behandlung bereitgestellt werden kann und gleichzeitig eine hohe Therapiesicherheit durch den regulierbaren Druck im Innenbereich des Oberschenkels gegeben ist.

## Patentansprüche

1. Kompressionsstrumpf aus einem elastischen Material mit einem an dem Kompressionsstrumpf (10) befestigbaren Druckkörper (20), wobei der Kompressionsstrumpf (10) ein Schenkelstrumpf ist und an seinem an einer Oberschenkelinnenseite eines Trägers zu liegen kommenden Bereich (13) eine Tasche (16) aufweist, in die der Druckkörper (20) entnehmbar einbringbar ist, **dadurch gekennzeichnet, dass** der Druck des Kompressionsstrumpfes (10) im Bereich außerhalb des Druckkörpers (20) im Wesentlichen unabhängig von der Einbringung oder Befüllung des Druckkörpers ist.

2. Kompressionsstrumpf nach Anspruch 1, **dadurch gekennzeichnet, dass** der Druckkörper (20) befüllbar und entleerbar ist, insbesondere mit einem Gas oder einer Flüssigkeit, vorzugsweise Luft oder Wasser.

3. Kompressionsstrumpf nach Anspruch 1 oder 2, **dadurch gekennzeichnet, dass** der Druckkörper (20) eine längliche Form besitzt, wobei dessen Längsachse am Oberschenkel eines Trägers von proximal nach distal verläuft.

4. Kompressionsstrumpf nach einem der Ansprüche 1 bis 3, **dadurch gekennzeichnet, dass** das elastische Material ein Gewebe, Gewirke oder Gestricke, vorzugsweise ein Rundgestricke ist.

5. Kompressionsstrumpf nach einem der vorangehenden Ansprüche, **dadurch gekennzeichnet, dass** der Kompressionsstrumpf (10) ohne Druckkörper (20) ein Kompressionsstrumpf (10) mit einem Druckverlauf nach RAL-GZ 387 oder Referentiel techniques des ortheses elastiques de contention des membres (ASQUAL) ist und insbesondere der jeweiligen Kompressionsklassen KKL II bzw. K1. 2.

6. Kompressionsstrumpf nach einem der vorangehenden Ansprüche, **dadurch gekennzeichnet, dass** unterhalb des Druckkörpers (20) der Druck 15-60 mmHg und insbesondere 20-45 mmHg über dem Druck des Kompressionsstrumpfes (10) an dieser Stelle ohne Druckkörper (20) liegt.

7. Kompressionsstrumpf nach einem der vorangehenden Ansprüche, **dadurch gekennzeichnet, dass** der Druckkörper (20) über der Vena saphena magna zu liegen kommt bei angezogenem Kompressionsstrumpf (10).

8. Kompressionsstrumpf nach einem der vorangehenden Ansprüche, **dadurch gekennzeichnet, dass** der Druckkörper (20) eine Erstreckung von unterhalb des Bündchens (18) bis wenigstens in den Bereich oberhalb des Knies (19) eines Trägers aufweist.

9. Kompressionsstrumpf nach einem der vorangehenden Ansprüche, **dadurch gekennzeichnet, dass** der Kompressionsstrumpf (10) am Oberschenkel durch ein an der Innenseite eines Bündchens (18) angeordneten Haftbands gegen Rutschen nach distal gesichert ist, wobei das Haftband eine die Haftung zwischen dem Bein des Träger und dem Kompressionsstrumpf (10) erhöhende Beschichtung aufweist.

10. Kompressionsstrumpf nach Anspruch 9, **dadurch gekennzeichnet, dass** die Beschichtung in Mustern, insbesondere Gittern oder in Umfangsrichtung verlaufenden Streifen oder Kalotten aufgebracht ist.

11. Kompressionsstrumpf nach einem der vorangehenden Ansprüche, **dadurch gekennzeichnet, dass** das Bündchen (18) einen von distal nach proximal an der Oberschenkelinnen und/oder -außenseite schrägen Verlauf von posterior nach anterior besitzt, wobei dieser Verlauf gekrümmt oder ungekrümmt ist.

12. Set bestehend aus einem Kompressionsstrumpf aus einem elastischen Material, wobei der Kompressionsstrumpf (10) ein Schenkelstrumpf ist und an seinem an einer Oberschenkelinnenseite eines Trägers zu liegen kommenden Bereich (13) eine Tasche (16) aufweist sowie einem an die Tasche (16) angepassten Druckkörper (20), der in die Tasche entnehmbar einbringbar ist, **dadurch gekennzeichnet, dass** der Druck des Kompressionsstrumpfes (10) im Bereich außerhalb des Druckkörpers (20) im Wesentlichen unabhängig von der Einbringung oder Befüllung des Druckkörpers ist.

13. Set nach Anspruch 12, **dadurch gekennzeichnet, dass** der Druckkörper (20) befüll- und entleerbar ist.

## Claims

1. Compression hose consisting of an elastic material with a pressure hull (20) that can be fastened to the compression hose (10), whereby the compression hose (10) is a thigh hose and at its section (13) that comes to bear at an upper inner side of the thigh of a wearer has a pocket (16), into which the pressure hull (20) can be inserted removable, **characterized in that** the pressure of the compression hose (10) in the section exterior to the pressure hull (20) is essentially independent of the insertion or filling of the pressure hull.

2. Compression hose according to claim 1, **characterized in that** the pressure hull (20) can be filled and emptied, in particular with a gas or a fluid, preferably air or water.

3. Compression hose according to claim 1 or 2, **characterized in that** the pressure hull (20) has an elongated form, whereby its longitudinal axis extends from proximal to distal on the upper thigh of a wearer.

4. Compression hose according to one of claims 1 to 3, **characterized in that** the elastic material is a cloth, knitted fabric or knotted fabric, preferably a fabric knitted in the round.

5. Compression hose according to one of the preceding claims, **characterized in that** the compression hose (10) without pressure hull (20) is a compression hose (10) with a pressure pattern according to RAL-GZ 387 or Referential techniques des ortheses elastiques de contention des membres (ASQUAL), and in particular of the respective compression classes KKL II or Kl. 2.

6. Compression hose according to one of the preceding claims, **characterized in that** underneath the pressure hull (20) the pressure is 15 - 60 mmHg, and in particular 20 - 45 mmHg above the pressure of the compression hose (10) at this position without pressure hull (20).

7. Compression hose according to one of the preceding claims, **characterized in that** the pressure hull (20) comes to bear above the vena saphena magna when the compression hose (10) has been put on.

8. Compression hose according to one of the preceding claims, **characterized in that** the pressure hull (20) has an extension from underneath the cuff (18) up to at least into the section above the knee (19) of a wearer.

9. Compression hose according to one of the preceding claims, **characterized in that** the compression hose (10) is secured against sliding in the distal direction at the upper thigh by an adhesive band located at the inner side of a cuff (18), whereby the adhesive band has a coating that elevates the adhesion between the leg of the carrier and the compression hose (10).

10. Compression hose according to claim 9, **characterized in that** the coating is applied in patterns, in particular grids, or in strips or calottes that extend in the direction of the circumference.

11. Compression hose according to one of the preceding claims, **characterized in that** the cuff (18) has a progression from distal to proximal at the upper thigh inner side and/or upper thigh outer side inclined that extends at an incline from posterior toward anterior, whereby this progression is curved or not curved.

12. Set consisting of a compression hose made of an elastic material, whereby the compression hose (10) is a thigh hose and at its section (13) that comes to bear at an upper inner side of the thigh of a wearer has a pocket (16), as well as a pressure hull (20) that is adapted to the pocket (16), which can be removed from and inserted into the pocket, **characterized in that** the pressure of the compression hose (10) in the range outside of the pressure hull (20) is essentially independent of the insertion or filling of the pressure hull.

13. Set according to claim 12, **characterized in that** the pressure hull (20) can be filled and emptied.

## Revendications

1. Bas de compression en matériau élastique comprenant un corps de pression (20) pouvant se fixer au bas de compression (10), le bas de compression (10) étant un bas pour cuisse et présentant, au niveau de sa zone (13) venant reposer sur une partie intérieure de la cuisse d'un porteur, une poche (16), dans laquelle on peut mettre ou retirer le corps de pression (20), **caractérisé en ce que** la pression du bas de compression (10) dans la zone située à l'extérieur du corps de pression (20) est essentiellement indépendante de l'apport ou du remplissage du corps de pression.

2. Bas de compression selon la revendication 1, **caractérisé en ce que** le corps de pression (20) peut être rempli et vidé, en particulier avec un gaz ou un liquide, de préférence de l'air ou de l'eau.

3. Bas de compression selon la revendication 1 ou 2, **caractérisé en ce que** le corps de pression (20) possède une forme allongée, l'axe longitudinal de ce dernier s'étendant au niveau de la cuisse du porteur du proximal vers le distal.

4. Bas de compression selon l'une des revendications 1 à 3, **caractérisé en ce que** le matériau élastique est un tissu, un maillage ou un tricotage, de préférence un tricotage rond.

5. Bas de compression selon l'une des revendications précédentes, **caractérisé en ce que** le bas de compression (10) sans corps de pression (20) est un bas de compression (10) avec un profil de pression suivant RAL-GZ 387 ou le Référentiel technique des orthèses élastiques de contention des membres (ASQUAL) et en particulier des classes de compression KKL II ou Kl. 2.

6. Bas de compression selon l'une des revendications précédentes, **caractérisé en ce que**, sous le corps de pression (20), la pression est de 15-60 mmHg et en particulier 20-45 mmHg supérieure à la pression du bas de compression (10) à ce même endroit sans le corps de pression (20).

7. Bas de compression selon l'une des revendications précédentes, **caractérisé en ce que** le corps de pression (20) vient reposer, lorsque le bas de compression (10) est enfilé, au-dessus de la grande veine saphène.

8. Bas de compression selon l'une des revendications précédentes, **caractérisé en ce que** le corps de pression (20) s'étend depuis sous le bord (18) jusqu'au moins dans la zone venant au-dessus du genou (19) d'un porteur.

9. Bas de compression selon l'une des revendications précédentes, **caractérisé en ce que** le bas de compression (10) est maintenu sur la cuisse contre tout glissement vers le distal par le biais d'une bande adhésive agencée sur le côté intérieur du bord (18), la bande adhésive présentant un revêtement augmentant l'adhésion entre la jambe du porteur et le bas de compression (10).

10. Bas de compression selon la revendication 9, **caractérisé en ce que** le revêtement est appliqué dans des dessins, en particulier dans des motifs quadrillés ou dans des bandes ou calottes s'étendant dans le sens circonférentiel.

11. Bas de compression selon l'une des revendications précédentes, **caractérisé en ce que** le bord (18) présente, de l'arrière vers l'avant, un profil oblique du distal vers le proximal au niveau de la partie intérieure et/ou extérieure de la cuisse, ce profil étant courbé ou non courbé.

12. Ensemble composé d'un bas de compression en matériau élastique, le bas de compression (10) étant un bas pour cuisse et présentant, au niveau de sa zone (13) venant reposer sur un côté intérieur de la cuisse d'un porteur, une poche (16) et composé également d'un corps de pression (20) adapté à la poche (16), lequel corps de pression peut être mis ou enlevé de la poche, **caractérisé en ce que** la pression du bas de compression (10) dans la zone située en dehors du corps de pression (20) est essentiellement indépendante de l'apport ou du remplissage du corps de pression.

13. Ensemble selon la revendication 12, **caractérisé en ce que** le corps de pression (20) peut être rempli et vidé.
